# EUROPEAN PATENT APPLICATION

(11) **EP 4 318 495 A1**
(43) Date of publication of application: **07.02.2024**
(21) Application number: 22778731.4
(22) Date of filing: 24.03.2022
(51) Int. Cl.: G16H 50/30

(54) **ELECTRONIC DEVICE AND BIOLOGICAL DETECTION CONTROL METHOD AND APPARATUS**

(30) Priority: 30.03.2021 CN 202110341286
(71) Applicant: Vivo Mobile Communication Co., Ltd., Dongguan, Guangdong 523863 (CN)
(72) Inventor: FU, Conghua, Dongguan, Guangdong 523863 (CN); YE, Jinshan, Dongguan, Guangdong 523863 (CN)
(74) Representative: Lavoix
(86) International application number: PCT/CN2022/082736
(87) International publication number: WO 2022/206548

(57) **Abstract**

This application discloses an electronic device, and a biological detection control method and apparatus, and relates to the field of biological detection technologies. The electronic device includes: a packaging layer, on which a light-transmitting part is provided; a fingerprint identification unit and a pulse wave detection unit disposed within the packaging layer; and a substrate, the fingerprint identification unit and the pulse wave detection unit being disposed on the substrate and fixedly connected to the substrate, where the pulse wave detection unit directly faces the light-transmitting part.

## Description

### CROSS-REFERENCE TO RELATED APPLICATIONS

This application claims priority to Chinese Patent Application No. 202110341286.X filed on March 30, 2021, which is incorporated herein by reference in its entirety.

### TECHNICAL FIELD

This application relates to the field of biological detection technologies, and in particular, to an electronic device, and a biological detection control method and apparatus.

### BACKGROUND

Accompanied with the continuous development of mobile communication technologies, intelligent electronic devices (for example, mobile phones) are becoming increasingly popular. Application of the fingerprint identification technology on smartphones has been popularized, and some terminals are provided with under-screen fingerprint identification sensors. Moreover, with the improvement of quality of life, people pay more attention to health, and outdoor running, swimming, indoor fitness, and other sports methods have gradually become a part of people's daily life. People increasingly hope to monitor data related to their bodies at any time by using portable intelligent devices, in order to learn about their health status.

At present, some terminals have implemented a heart rate detection function on under-screen fingerprint identification sensors, but due to a low quality of pulse waves acquired by using an optical under-screen fingerprint identification technology, it is still impossible to measure health status such as blood oxygen and blood pressure by using the under-screen fingerprint identification sensors, and during acquisition of pulse waves by using the optical under-screen fingerprint identification sensors, an organic light-emitting diode (organic light-emitting diode, OLED) display screen is needed, to continuously expose at the highest brightness, which on one hand, affects the lifespan of the OLED and on the other hand, has a high power consumption and therefore is not suitable for applications for long-term measurement of, such as, blood oxygen and blood pressure.

### SUMMARY

An objective of embodiments of this application is to provide an electronic device, and a biological detection control method and apparatus, which can solve the problem of a low quality of pulse waves acquired by using an existing optical under-screen fingerprint identification sensor and incapability in implementing measurement of blood oxygen and blood pressure.

To resolve the foregoing technical problems, this application is implemented as follows:
According to a first aspect, an embodiment of this application provides a biological detection module, including:
a packaging layer, on which a light-transmitting part is provided;
a fingerprint identification unit and a pulse wave detection unit disposed within the packaging layer; and
a substrate, the fingerprint identification unit and the pulse wave detection unit being disposed on the substrate and fixedly connected to the substrate, where
the pulse wave detection unit directly faces the light-transmitting part.

According to a second aspect, an embodiment of this application further provides an electronic device, including the biological detection module according to the first aspect; and
a housing, the biological detection module being disposed on the housing.

According to a third aspect, an embodiment of this application further provides a biological detection control method, including:
receiving a first input by a first user on a biological detection module, where the biological detection module includes a fingerprint identification unit and a pulse wave detection unit;
in response to the first input, obtaining a finger pressing state of the first user obtained by the fingerprint identification unit and pulse wave data acquired by the pulse wave detection unit;
determining the pulse wave data as target pulse wave data in a case that the finger pressing state of the first user meets a pulse wave acquisition trigger condition; and
obtaining health indicator data of the first user based on the target pulse wave data.

According to an fourth aspect, an embodiment of this application further provides a biological detection control apparatus, including:
a receiving module, configured to receive a first input by a first user on a biological detection module, where the biological detection module includes a fingerprint identification unit and a pulse wave detection unit;
a first obtaining module, configured to in response to the first input, obtain a finger pressing state of the first user obtained by the fingerprint identification unit and pulse wave data acquired by the pulse wave detection unit;
a first processing module, configured to determine the pulse wave data as target pulse wave data in a case that the finger pressing state of the first user meets a pulse wave acquisition trigger condition; and
a second processing module, configured to obtain health indicator data of the first user based on the target pulse wave data.

According to a fifth aspect, an embodiment of this application provides an electronic device, including a processor, a memory, and a program or instructions stored on the memory and executable by the processor, where the program or instructions, when executed by the processor, implement the steps of implementing the biological detection control method according to the third aspect.

According to a sixth aspect, an embodiment of this application further provides a readable storage medium, storing a program or instructions, where the program or instructions, when executed by a processor, implement the steps of implementing the biological detection control method according to the third aspect.

According to a seventh aspect, an embodiment of this application provides a chip, where the chip includes a processor and a communication interface, the communication interface is coupled to the processor, and the processor is configured to run a program or instructions to implement the biological detection control method according to the third aspect.

According to an eighth aspect, provided is a computer program product, where the computer program product is stored in a non-volatile storage medium, and the computer program product, when executed by at least one processor, implements the biological detection control method according to the third aspect.

In this embodiment of this application, by using the packaging layer provided with the light-transmitting part, the fingerprint identification unit and the pulse wave detection unit disposed within the packaging layer, and the substrate, the fingerprint identification unit and the pulse wave detection unit being disposed on the substrate and fixedly connected to the substrate, where the pulse wave detection unit directly faces the light-transmitting part, the fingerprint identification unit and the pulse wave detection unit are integrated into one biological detection module, which is subsequently disposed on a housing of an electronic device, so that a pulse wave detection function can be achieved based on non-under-screen fingerprint identification, thereby improving the quality of pulse waves and further implementing measurement of health indicators such as heart rate, blood oxygen, and blood pressure.

### BRIEF DESCRIPTION OF THE DRAWINGS

FIG. 1 is a first schematic structural diagram of a biological detection module according to an embodiment of this application;
FIG. 2 is a second schematic structural diagram of a biological detection module according to an embodiment of this application;
FIG. 3 is a third schematic structural diagram of a biological detection module according to an embodiment of this application;
FIG. 4 is a fourth schematic structural diagram of a biological detection module according to an embodiment of this application;
FIG. 5 is a fifth schematic structural diagram of a biological detection module according to an embodiment of this application;
FIG. 6 is a sixth schematic structural diagram of a biological detection module according to an embodiment of this application;
FIG. 7 is a seventh schematic structural diagram of a biological detection module according to an embodiment of this application;
FIG. 8 is an eighth schematic structural diagram of a biological detection module according to an embodiment of this application;
FIG. 9 is a first schematic structural diagram of an electronic device according to an embodiment of this application;
FIG. 10 is a first schematic flowchart of a biological detection control method according to an embodiment of this application;
FIG. 11 is a first schematic diagram of comparison between fingerprint information acquired by a biological detection module and a fingerprint template according to an embodiment of this application;
FIG. 12 is second schematic diagram of comparison between fingerprint information acquired by a biological detection module and a fingerprint template according to an embodiment of this application;
FIG. 13 is a third schematic diagram of comparison between fingerprint information acquired by a biological detection module and a fingerprint template according to an embodiment of this application;
FIG. 14 is a fourth schematic diagram of comparison between fingerprint information acquired by a biological detection module and a fingerprint template according to an embodiment of this application;
FIG. 15 is a second schematic flowchart of a biological detection control method according to an embodiment of this application;
FIG. 16 is a third schematic flowchart of a biological detection control method according to an embodiment of this application;
FIG. 17 is a fourth schematic flowchart of a biological detection control method according to an embodiment of this application;
FIG. 18 is a schematic structural diagram of a biological detection control apparatus according to an embodiment of this application;
FIG. 19 is a second schematic structural diagram of an electronic device according to an embodiment of this application; and
FIG. 20 is a schematic structural diagram of a hardware structure of an electronic device according to an embodiment of this application.

### DETAILED DESCRIPTION

The technical solutions in embodiments of this application are clearly and completely described in the following with reference to the accompanying drawings in the embodiments of this application. Apparently, the described embodiments are merely some rather than all of the embodiments of this application. All other embodiments obtained by a person of ordinary skill in the art based on the embodiments of this application without making creative efforts shall fall within the protection scope of this application.

The specification and claims of this application, and terms "first" and "second" are used to distinguish similar objects, but are unnecessarily used to describe a specific sequence or order. It is to be understood that data used in this way is exchangeable in a proper case, so that the embodiments of this application described herein can be implemented in an order different from the order shown or described herein, and in addition, the objects distinguished by "first" and "second" are generally one type, and the number of the objects is not limited, for example, there may be one first object, or a plurality of first objects. In addition, "and/or" used in this specification and the claims represents at least one of the connected objects, and the character "/" generally indicates that the associated objects are in an "or" relationship.

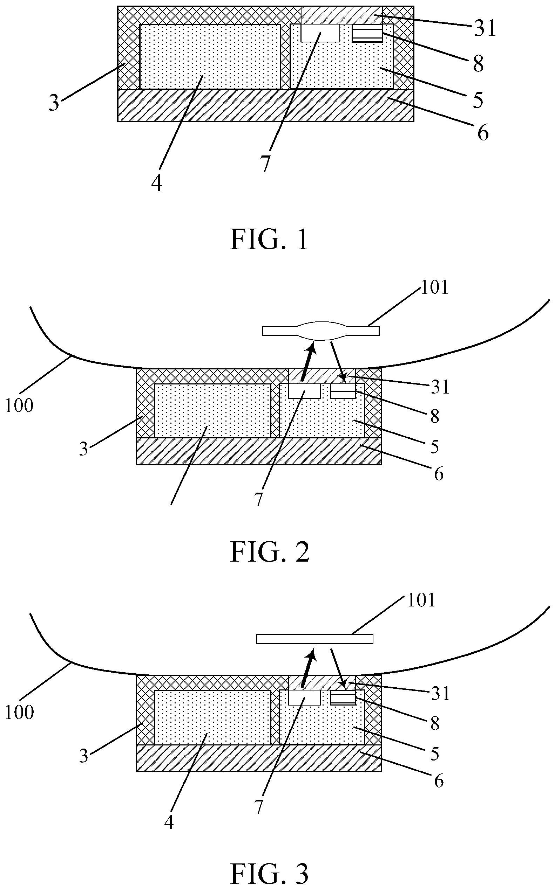

FIG. 1 to FIG. 8 are schematic structural diagrams of a biological detection module provided in the embodiments of this application. The biological detection module includes: a packaging layer 3, on which a light-transmitting part 31 is provided; a fingerprint identification unit 4 and a pulse wave detection unit 5 disposed within the packaging layer 3; and a substrate 6, the fingerprint identification unit 4 and the pulse wave detection unit 5 being disposed on the substrate 6 and fixedly connected to the substrate 6, where the pulse wave detection unit 5 directly faces the light-transmitting part 31.

It may also be understood that an orthographic projection of the pulse wave detection unit 5 on the packaging layer 3 is in a region where the light-transmitting part 31 is located.

Here, the fingerprint identification unit 4 is configured to acquire fingerprint data of a user finger pressed on a surface of the biological detection module. The working principle is based on a capacitance principle, specifically, acquiring a fingerprint pattern of the user finger.

The pulse wave detection unit 5 is configured to acquire pulse waves of blood vessels inside the user finger pressed on the surface of the biological detection module. The working principle is based on an optical principle, specifically, acquiring a pulse wave signal by acquiring a blood volume change of an arterial blood vessel inside the user finger.

It should be noted that the fingerprint identification unit 4 and the pulse wave detection unit 5 may work simultaneously or may work in a time-division manner.

Here, the biological detection module integrated with the fingerprint identification unit 4 and the pulse wave detection unit 5 may acquire both fingerprint data of the user finger and pulse wave data of the user finger.

Here, both the fingerprint identification unit 4 and the pulse wave detection unit 5 are fastened on the substrate 6 and electrically connected to the substrate 6 through components such as a solder pad, a solder ball, and a gold wire.

The packaging layer 3 is provided with the light-transmitting part 31 and therefore light emitted by the pulse wave detection unit 5 passes through the light-transmitting part 31 and reaches the user finger, is reflected by the user finger, and then returns to the pulse wave detection unit 5 through the light-transmitting part 31, thereby implementing acquisition of a pulse wave signal. Moreover, due to the extreme sensitivity of the pulse wave detection unit 5 to static electricity, the pulse wave detection unit 5, when directly exposed on an external surface, is apt to be disturbed or even broken down by external static electricity. In addition to protecting the pulse wave detection unit 5, the packaging layer 3 also provides an electrostatic isolation function for the pulse wave detection unit 5. In addition, the light-transmitting part 31 of the packaging layer 3 can further ensure that emitted light and reflected light can pass through efficiently. It should be noted that generally, the light-transmitting part 31 of the packaging layer 3 adopts a material having a relatively high transmittance within the wavelength range of light emitted by the pulse wave detection unit 5.

Optionally, the pulse wave detection unit 5 includes a light emitting component 7 and a light receiving component 8; and light emitted by the light emitting component 7 passes through the light-transmitting part 31, to reach a finger of a user to be detected, and is reflected by the finger of the user to be detected, so as to be received by the light receiving component 8.

Here, optionally, the light emitting component 7 is a light-emitting diode (Light-Emitting Diode, LED). The number of light-emitting diodes LEDs in this embodiment of this application is not limited, but is typically greater than one. More commonly, the LEDs may emit two to three colors of light, for example, red light, green light, and infrared light.

Optionally, the light receiving component 8 is a photo-diode (Photo-Diode, PD). The PD may receive all colors of light emitted by the LEDs. To improve the sensitivity and signal quality of receiving light, there may be two or more PDs. For ease of explanation, in this embodiment of this application, one LED and one PD are used for illustration.

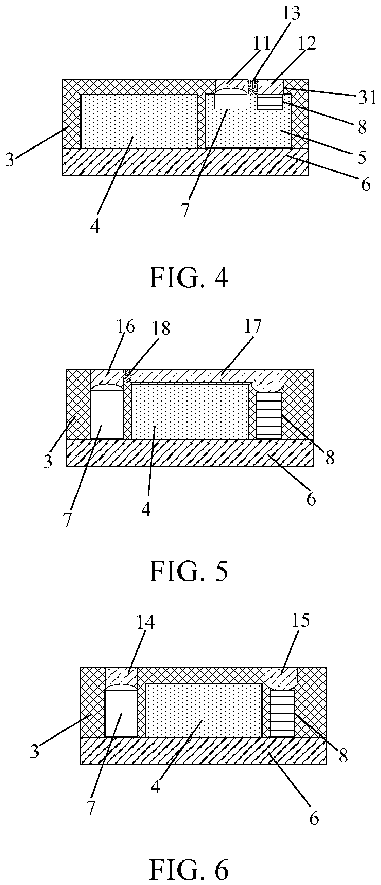

Specifically, in a case that the light emitted by the light emitting component 7 (for example, the LED) is irradiated on a user finger 100, tissues such as arterial blood, a muscle, and skin of the user finger 100 absorb part of the light and reflect part of the light, and the reflected light is captured by the light receiving component 8 (for example, the PD) and converted into an electrical signal. Due to contraction and relaxation of a heart, a large amount of blood is pumped into an arterial blood vessel 101 during cardiac contraction, to cause an increase in the volume, as shown in FIG. 3; and the blood in the arterial blood vessel 101 flows back to the heart during cardiac relaxation, to cause a decrease in the volume, as shown in FIG. 4. In other words, reflected light rays captured by the light receiving component 8 decrease during cardiac contraction and increase during cardiac relaxation, which presents a periodic pulsating trend in the electrical signal. This pulsation signal is an original pulse wave signal.

In an optional implementation, as shown in FIG. 5, an inner surface of the light-transmitting part 31 and corresponding to the light emitting component 7 has a light gathering property.

Here, the inner surface of the light-transmitting part 31 and corresponding to the light emitting component 7 in this implementation has a light gathering property, which can effectively gather the light emitted by the light emitting component 7, to control the emitted light to transmit within a desired direction range. For example, the inner surface of the light-transmitting part 31 and corresponding to the light emitting component 7 is a convex surface bent in a direction approaching an outer surface of the light-transmitting part 31, or the inner surface of the light-transmitting part 31 and corresponding to the light emitting component 7 is a concave-convex surface having a light gathering property, for example, a concave-convex surface designed as a Fresnel pattern, which is not specifically limited herein. It should be noted that in a case that the emission direction range is excessively large, leakage of light to the outside of the finger is apt to be caused, which reduces the energy efficiency, and the emitted light is apt to be introduced into the light-transmitting part 31 of the corresponding light receiving component 8, which causes crosstalk. This implementation can reduce optical crosstalk between the light emitting component 7 and the light receiving component 8.

Here, the light-transmitting part 31 corresponding to the light receiving component 8 has a collimating effect, to control to receive the reflected light within a desired receiving direction range. It should be noted that by using an excessively large range of receiving direction range, ambient light interference is apt to be introduced, and by using an excessively small receiving direction range, reflected light cannot be fully utilized, which causes insufficient sensitivity.

Optionally, the light emitting component 7 and the light receiving component 8 are located on a same side of the fingerprint identification unit 4, as shown in FIG. 1 and FIG. 3 to FIG. 5.

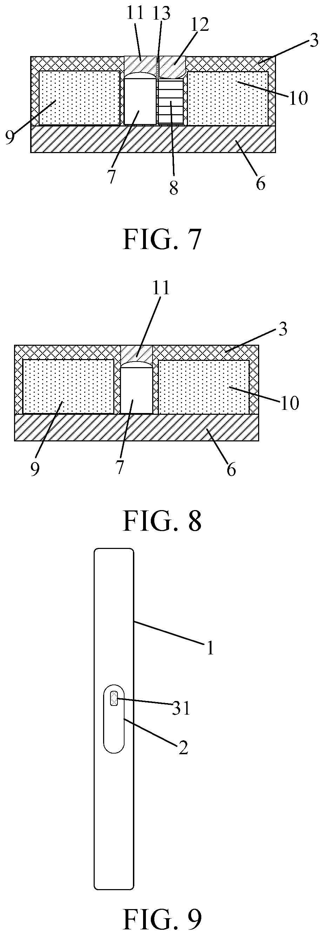

Alternatively, the light emitting component 7 and the light receiving component 8 are respectively located on two opposite sides of the fingerprint identification unit 4, as shown in FIG. 6 and FIG. 7.

Here, in a case that the light emitting component 7 and the light receiving component 8 are located on the two opposite sides of the fingerprint identification unit 4, optical isolation of physical components is implemented by using the fingerprint identification unit 4, thereby solving the problem of the optical crosstalk between the light emitting component 7 and the light receiving component 8.

Alternatively, in a case that the fingerprint identification unit 4 includes a first fingerprint identification unit 9 and a second fingerprint identification unit 10, the light emitting component 7 and the light receiving component 8 are located between the first fingerprint identification unit 9 and the second fingerprint identification unit 10, as shown in FIG. 8 and FIG. 9.

Here, the fingerprint identification unit 4 includes a first fingerprint identification unit 9 and a second fingerprint identification unit 10, which may be distributed in discontinuous regions on the substrate 6. It should be noted that the electronic device may independently use a fingerprint image acquired by the first fingerprint identification unit 9 or the second fingerprint identification unit 10, or may stitch and merge fingerprint images acquired by the first fingerprint identification unit 9 and the second fingerprint identification unit 10, to form a fingerprint image.

It should be noted that the light emitting component 7 and the light receiving component 8 may be arranged between the first fingerprint identification unit 9 and the second fingerprint identification unit 10 in a direction parallel to a long edge direction of the biological detection module, as shown in FIG. 8, that is, arranged horizontally; or may be arranged in a direction parallel to a wide edge direction of the biological detection module, that is, arranged longitudinally, as shown in FIG. 9. In this way, the pulse wave detection unit 5 is concentrated in a central region of the biological detection module, which on one hand, can better align with a pulp of the user finger, so that the electronic device can easily obtain a high-quality pulse wave signal; and on the other hand, can minimize the impact on appearance.

Based on a positional relationship between the light emitting component 7 and the fingerprint identification unit 4 and between the light receiving component 8 and the fingerprint identification unit 4, in an optional implementation, in a case that the light emitting component 7 and the light receiving component 8 are located on the same side of the fingerprint identification unit 4, or in a case that the light emitting component 7 and the light receiving component 8 are located between the first fingerprint identification unit 9 and the second fingerprint identification unit 10, both transmittance of the first light-transmitting portion 11 and transmittance of the second light-transmitting portion 12 are greater than that of a third light-transmitting portion 13, where the first light-transmitting portion 11 is a portion where the light-transmitting part 31 corresponds to the light emitting component 7, the second light-transmitting portion 12 is a portion where the light-transmitting part 31 corresponds to the light receiving component 8, and the third light-transmitting portion 13 is a portion where the light-transmitting part 31 connects the first light-transmitting portion 11 and the second light-transmitting portion 12.

Here, as shown in FIG. 5 and FIG. 8, both the transmittance of the first light-transmitting portions 11 and the transmittance of the second light-transmitting portion 12 are greater than that of the third light-transmitting portion 13. The purpose is to reduce the optical crosstalk between the light emitting component 7 and the light receiving component 8, thereby improving the pulse wave quality.

In an optional implementation, in a case that the light emitting component 7 and the light receiving component 8 are respectively located on two opposite sides of the fingerprint identification unit 4, the light-transmitting part 31 covers the light emitting component 7, the light receiving component 8, and the fingerprint identification unit 4, as shown in FIG. 6, which may also be understood as the light emitting component 7, the light receiving component 8, and the fingerprint identification unit 4 are located in an orthographic projection region of the light-transmitting part 31 on the substrate 6.

In another optional implementation, in a case that the light emitting component 7 and the light receiving component 8 are respectively located on two opposite sides of the fingerprint identification unit 4, the light-transmitting part 31 includes a fourth light-transmitting portion 14 and a fifth light-transmitting portion 15, and the light emitting component 7 directly faces the fourth light-transmitting portion 14, which may also be understood as that the light emitting component 7 is located in an orthographic projection region of the fourth light-transmitting portion 14 on the substrate 6, and the light receiving component 8 directly faces the fifth light-transmitting portion 15, which may also be understood as that the light receiving component 8 is located in an orthographic projection region of the fifth light-transmitting portion 15 on the substrate 6, as shown in FIG. 7.

It should be noted that as shown in FIG. 6, the light-transmitting part 31 can completely cover an orthographic projection of the fingerprint identification unit 4 thereon, and in addition, cover an orthographic projection of the light emitting component 7 and an orthographic projection of the light receiving component 8 thereon. The light-transmitting part 31 may also be configured discontinuously, as shown in FIG. 7.

Based on the case in which the light-transmitting part 31 covers the light emitting component 7, the light receiving component 8, and the fingerprint identification unit 4, in an optional implementation, as shown in FIG. 6, both transmittance of a sixth light-transmitting portion 16 and transmittance of a seventh light-transmitting portion 17 are greater than that of an eighth light-transmitting portion 18, where the sixth light-transmitting portion 16 is a portion where the light-transmitting part 31 corresponds to the light emitting component 7, the seventh light-transmitting portion 17 is a portion where the light-transmitting part 31 corresponds to the light receiving component 8 and the fingerprint identification unit 4, and the eighth light-transmitting portion 18 is a portion where the light-transmitting part 31 connects the sixth light-transmitting portion 16 and the seventh light-transmitting portion 17.

In this implementation, the light-transmitting part 31 can completely cover an orthographic projection of the fingerprint identification unit 4 thereon, and in addition, cover an orthographic projection of the light emitting component 7 and an orthographic projection of the light receiving component 8 thereon; moreover, both transmittance of the eighth light-transmitting portion 18 between the light emitting component 7 and the light receiving component 8 is less than transmittance of the other two portions of the light-transmitting part 31, so as to achieve good optical isolation and reduce crosstalk.

It should be noted that in this implementation, an area of the sixth light-transmitting portion 16 is smaller than an area of the seventh light-transmitting portion 17, that is, an area of the light-transmitting part 31 corresponding to the light emitting component 7 is smaller than an area of the light-transmitting part 31 corresponding to the light receiving component 8. The reason is that the light emitting component 7 may typically adjust the transmit power to meet different use scenarios, the sensitivity of the light receiving component 8 is fixed, and by increasing the transmissive portion 31 (that is, the seventh transmissive portion 17) on the side of the light receiving component 8, an amount of light received by the light receiving component 8 can be increased. In this way, the detection performance of the light receiving component 8 in different environments (for example, strong light environments) at a relatively low transmit power can be improved.

Optionally, the light-transmitting part 31 is made of a hard optical sheet material; or, the light-transmitting part 31 is provided with an ink coating having light transmittance.

Optionally, the light-transmitting part 31 is a lens.

It should be noted that by enabling the light-transmitting part 31 to be a lens, constituent components (mainly the light emitting component 7 and the light receiving component 8) of the pulse wave detection unit 5 can be prevented from being seen from an external surface, which affects the appearance performance. Here, the lens is typically etched with patterns, for example, Fresnel patterns on a flashing light lens of a mobile phone, so that the constituent components of the pulse wave detection unit 5 cannot be seen from the external surface.

Here, it should be noted that the material of the lens has a high transmittance within the wavelength range of the light emitted by pulse wave detection unit 5, so as to ensure that the emitted light and the reflected light can pass through efficiently.

Optionally, an outer surface, of the light-transmitting part 31, directly facing the light receiving component 8 is provided with a filter layer, where the filter layer is configured to filter out light that enters the light receiving component 8 and exceeds a wavelength band of light to be received by the light receiving component 8.

Here, the outer surface of the light-transmitting part 31 is provided with the filter layer, to on one hand, prevent the constituent components of the pulse wave detection unit 5 from being seen from the external surface, and on the other hand, filter out the light that enters the light receiving component 8 and exceeds the wavelength band of the light to be received by the light receiving component 8, so as to ensure that the reflected light can pass through efficiently, thereby avoiding interference caused by other light.

In the biological detection module of this embodiment of this application, by using the packaging layer provided with the light-transmitting part, the fingerprint identification unit and the pulse wave detection unit disposed within the packaging layer, and the substrate, the fingerprint identification unit and the pulse wave detection unit being disposed on the substrate and fixedly connected to the substrate, where the pulse wave detection unit directly faces the light-transmitting part, the fingerprint identification unit and the pulse wave detection unit are integrated into one biological detection module, which is subsequently disposed on a housing of an electronic device, so that a pulse wave detection function can be achieved based on non-under-screen fingerprint identification, thereby improving the quality of pulse waves and further implementing measurement of health indicators such as heart rate, blood oxygen, and blood pressure.

An embodiment of this application further provides an electronic device, and as shown in FIG. 9, the electronic device includes the biological detection module 2 as described above; and a housing 1, the biological detection module 2 being disposed on the housing 1.

It should be noted that the biological detection module 2 is disposed on the housing 1 of the electronic device, and specifically, may be disposed on a border, a front housing, or a rear cover of the electronic device, which is not specifically limited herein. An outermost surface of the biological detection module 2 may be lower than an outer surface of the housing 1, that is, concave relative to the housing 1; or may be flush with an outer surface of the housing 1; or may be higher than an outer surface of the housing 1.

Due to the fact that the biological detection module 2 is disposed on the housing 1 of the electronic device, fingerprint identification of the fingerprint identification unit 4 is non-under-screen fingerprint identification, and fingerprint identification of the fingerprint identification unit 4 in this embodiment of this application is capacitive fingerprint identification.

It should be noted that the electronic device of this embodiment of this application further includes a main control unit and a display unit. The main control unit is typically a microprocessor-based computing and storage unit, which on one hand, controls the biological detection module to acquire a biological signal and process the biological signal into health indicator data, and on the other hand, presents the biological signal and/or the health indicator data in the display unit.

The biological detection module in this embodiment of this application may acquire both fingerprint information and pulse wave data. The biological detection module provides the fingerprint information to the main control unit, so that the main control unit processes the fingerprint information into a fingerprint image. By using the pre-acquired and saved fingerprint image, the main control unit may calculate a position, a direction, an area, and even a force by which a finger presses on the surface of the biological detection module each time.

The biological detection module provides the acquired pulse wave data to the main control unit, so that the main control unit processes the pulse wave data into a pulse wave waveform graph, blood oxygen saturation data, and blood pressure data, and displays the pulse wave waveform graph, the blood oxygen saturation data, and the blood pressure data on the display unit. By using preset threshold ranges, the main control unit may generate health alert information in a case that the blood oxygen saturation and the blood pressure data exceed the threshold ranges, and present the health alert information through the display unit.

The display unit is typically composed of display modules, functions of which are typically not only displaying, but also integrated with a touch control function, and is a main carrier for human-computer interaction between the electronic device and a user.

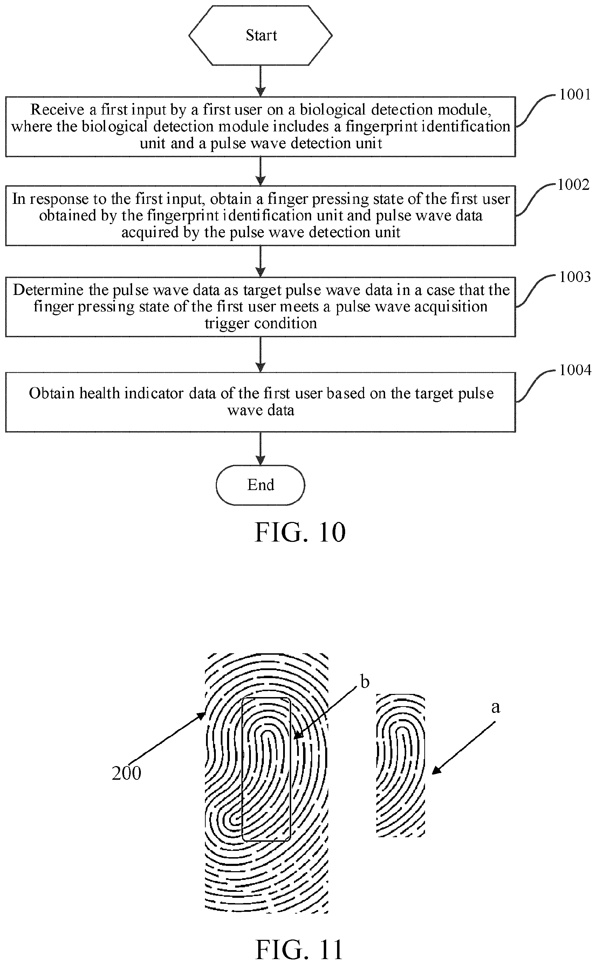

In the electronic device of this embodiment of this application, by disposing the biological detection module having the structure in the above embodiments on the housing of the electronic device, a pulse wave detection function can be implemented based on non-under-screen fingerprint identification, thereby improving the pulse wave quality and implementing measurement of health indicators such as a heart rate, blood oxygen, and blood pressure. As shown in FIG. 10, an embodiment of this application further provides a biological detection control method, which is applied to the electronic device according to the above embodiments. The method may include:
Step 1001: Receive a first input by a first user on a biological detection module, where the biological detection module includes a fingerprint identification unit and a pulse wave detection unit.

In this step, optionally, the first input is a press input.

Step 1002: In response to the first input, obtain a finger pressing state of the first user obtained by the fingerprint identification unit and pulse wave data acquired by the pulse wave detection unit.

Here, obtaining the finger pressing state of the first user obtained by the fingerprint identification unit specifically includes: obtaining fingerprint information acquired by the fingerprint identification unit, and determining the finger pressing state of the first user based on the fingerprint information.

In this step, optionally, the finger pressing state includes: a position at which, a direction in which, a contact area of which, and a force by which a finger presses.

It should be noted that typically, in a case that a user enables a fingerprint identification function, the user is required to enter a fingerprint template to be saved for comparison with a subsequently acquired fingerprint.

In this step, the fingerprint information acquired by the fingerprint identification unit is obtained, and is compared with the pre-saved fingerprint template, to determine the finger pressing state of the first user.

Specifically, by comparing the obtained fingerprint information of the first user with the pre-saved fingerprint template, a specific location, direction, and a size of an area of the first user finger currently in contact with the biological detection module can be obtained. In addition, a finger pressing force is positively correlated with the contact area (within a particular range), and the finger pressing force can be estimated based on a size of the fingerprint area.

Specific reasons are as follows:
in a case that the electronic device receives a press input by a user on the biological detection module, the fingerprint identification unit acquires a fingerprint once and saves the fingerprint as a first fingerprint; and by comparing the first fingerprint with the pre-saved fingerprint template, a finger pressing state of the user can be obtained.

In an ideal situation, the center of a finger pulp being pressed on the surface of the biological detection module is an ideal state, and in this case, a first fingerprint a has a quite high image overlap degree with a center area b of a fingerprint template 200, as shown in FIG. 11.

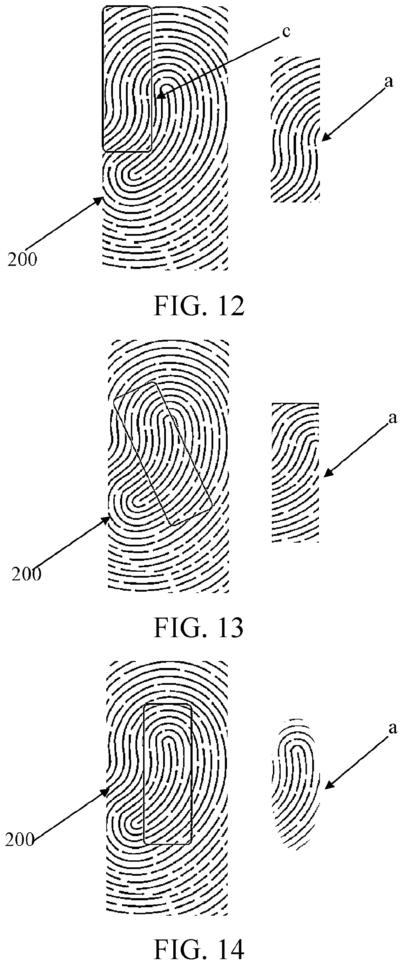

In a case that the contact position between the finger and the surface of the biological detection module deviates excessively far from the center, energy of reflected light, of the arterial blood vessel, received by the pulse wave detection unit is relatively small, resulting in a decrease in the quality of the pulse wave signal, which is manifested by a high degree of image overlap between the first fingerprint a and an edge region c of the fingerprint template 200, as shown in FIG. 12.

In a case that there is a significant change in the direction of contact between the finger and the surface of the biological detection module, as shown in FIG. 13, by comparing FIG. 13 and FIG. 11, the direction of the finger in FIG. 13 has significantly changed, and the quality of the pulse wave signal certainly needs to change. This situation is quite unfavorable for improving the accuracy of estimating health indicator data.

In a case that the finger pressing force is insufficient, the contact between the finger pulp and the surface of the biological detection module is insufficient, and the coupling between the pulse wave detection unit and the fingers is also insufficient, resulting in a decrease in the quality of the pulse wave signal, which is manifested by an excessively small image overlap area between the first fingerprint a and an edge position of the fingerprint template 200, as shown in FIG. 14. Here, boxes in FIG. 11 to FIG. 14 represent overlap regions between the first fingerprint and the fingerprint template.

It can be seen that by the foregoing method, during acquisition of the pulse wave signal, by comparing the first fingerprint with the fingerprint template, the finger pressing state can be determined, and whether the finger pressing state meets a requirement can also be determined.

Step 1003: Determine the pulse wave data as target pulse wave data in a case that the finger pressing state of the first user meets a pulse wave acquisition trigger condition.

Step 1004: Obtain health indicator data of the first user based on the target pulse wave data.

By the biological detection control method of this embodiment of this application, by receiving a first input by a first user on a biological detection module, where the biological detection module includes a fingerprint identification unit and a pulse wave detection unit, in response to the first input, obtaining a finger pressing state of the first user obtained by the fingerprint identification unit and pulse wave data acquired by the pulse wave detection unit, determining the pulse wave data as target pulse wave data in a case that the finger pressing state of the first user meets a pulse wave acquisition trigger condition, and obtaining health indicator data of the first user based on the target pulse wave data, a pulse wave detection function can be implemented, thereby improving the pulse wave quality and implementing measurement of health indicators such as a heart rate, blood oxygen, and blood pressure.

To further improve the pulse wave quality, to enable the electronic device to obtain high-quality pulse wave data and improve the accuracy of pulse wave detection, in an optional implementation, the step 1003 of the method of this embodiment of this application, determining the pulse wave data as target pulse wave data in a case that the finger pressing state of the first user meets a pulse wave acquisition trigger condition, includes one of the following:
controlling the pulse wave detection unit to acquire the pulse wave data and determining the pulse wave data as the target pulse wave data in a case that the finger pressing state of the first user meets a pulse wave acquisition trigger condition.

Here, the step of controlling the pulse wave detection unit to acquire pulse wave data in a case that the first user finger pressing state meets the pulse wave acquisition trigger condition is performed because in a case that a finger pressing position is offset excessively far (for example, a side of the finger pulp is in contact with the biological detection module), or the pressing force is excessively small (for example, the finger pulp is in insufficient contact with the biological detection module, and there is an excessively large amount of air on the interface), or a finger direction changes greatly in a process of acquiring a pulse wave, the quality of the acquired pulse wave data is affected, and the data quality has a great impact on the accuracy of subsequent calculation of health indicator data.

Here, the pulse wave acquisition trigger condition may be that the position of the finger press is in a preset region, the direction is in a preset direction, the contact area is greater than a preset first threshold, the force is greater than a preset second threshold, or the like.

Correspondingly, in this scenario, after controlling the pulse wave detection unit to acquire the pulse wave data and determining the pulse wave data as the target pulse wave data in a case that the finger pressing state of the first user meets a pulse wave acquisition trigger condition, this embodiment of this application may further include:
generating first prompt information, where the first prompt information is used for prompting the first user to start testing and to maintain a stable finger pressing state.

Here, the first prompt information can prompt the user to maintain the stable finger pressing state, so as to enable the electronic device to continuously obtain high-quality pulse wave data in a process in which the pulse wave detection unit acquires the pulse wave data.

Optionally, the method of this embodiment of this application further includes:
generating second prompt information in a case that the finger pressing state of the first user does not meet the pulse wave acquisition trigger condition, where the second prompt information is used for prompting the first user to adjust the finger pressing state.

Here, the second prompt information can prompt the user to adjust the finger pressing state, so as to enable the finger pressing state to meet the pulse wave acquisition trigger condition, thereby obtaining high-quality pulse wave data.

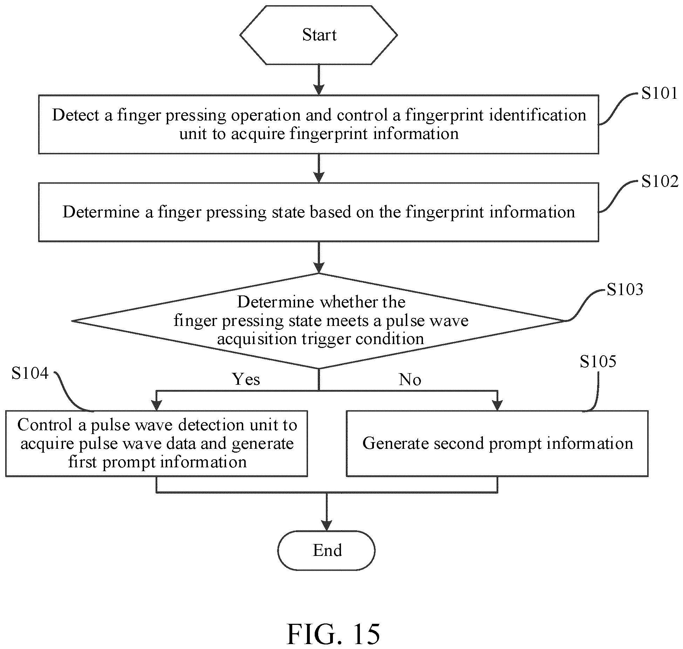

The following is an example corresponding to this scenario, specifically explaining the implementation process of the method in this embodiment of this application, as shown in FIG. 15.

S101: Detect a finger pressing operation and control a fingerprint identification unit to acquire fingerprint information.

S102: Determine a finger pressing state based on the fingerprint information.

S103: Determine whether the finger pressing state meets a pulse wave acquisition trigger condition;
if yes, perform step S104; or if not, perform step S105.

S104: Control a pulse wave detection unit to acquire pulse wave data and generate first prompt information.

Here, the first prompt information may be displayed on a display unit of the electronic device, to inform the user to keep the finger pressing state (for example: "The test has started. Please keep the finger pressing state stably").

S105: Generate second prompt information.

Here, the second prompt information may be displayed on the display unit of the electronic device, to guide the user to adjust the finger pressing state (for example: "The finger pressing force is too light. Please press a bit heavier").

The pulse wave detection unit is controlled to acquire the pulse wave data, determine whether the finger pressing state of the first user meets the pulse wave acquisition trigger condition in a process of acquiring the pulse wave data, and in a case that the pulse wave acquisition trigger condition is met, the pulse wave data is determined as the target pulse wave data.

Here, controlling the pulse wave detection unit to acquire the pulse wave data does not require any other restriction condition. However, to obtain high-quality pulse wave data, in a process of acquiring pulse wave data, the fingerprint identification unit periodically acquires fingerprint information of the first user, obtains a latest finger pressing state of the first user based on the fingerprint information of the first user, and determines whether the finger pressing state at this time meets the pulse wave acquisition trigger condition. In a case that the pulse wave acquisition trigger condition are met, it indicates that the finger pressing state is good, and the pulse wave detection unit is controlled to continuously acquire pulse wave data and determine pulse wave data obtained after the end of the acquisition as the target pulse wave data.

Optionally, after determining whether the finger pressing state of the first user meets the pulse wave acquisition trigger condition in the process of acquiring pulse wave data, the method of this embodiment of this application may further include:
in a case that the pulse wave acquisition trigger condition is not met, performing one of the following:
controlling the pulse wave detection unit to stop acquiring pulse wave data; or
generating third prompt information, where the third prompt information is used for instructing the first user to adjust the finger pressing state.

Here, after determining that the finger pressing state of the first user does not meet the pulse wave acquisition trigger condition in the process of acquiring pulse wave data, both controlling the pulse wave detection unit to stop acquiring pulse wave data, delete the previously acquired data, and perform detection again and generating the third prompt information for instructing the first user to adjust the finger pressing state are for obtaining high-quality pulse wave data.

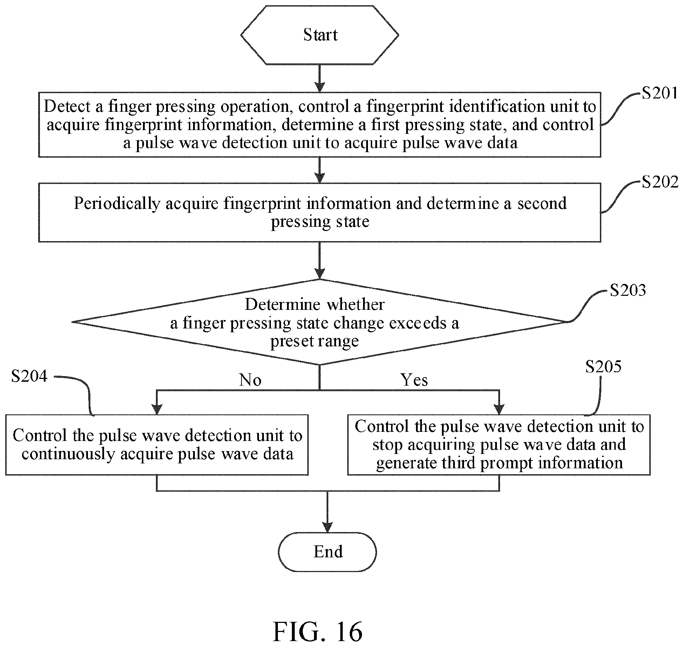

The following is an example corresponding to this scenario, specifically explaining the implementation process of the method in this embodiment of this application, as shown in FIG. 16.

S201: Detect a finger pressing operation, control a fingerprint identification unit to acquire fingerprint information, determine a first pressing state, and control a pulse wave detection unit to acquire pulse wave data.

S202: Periodically acquire fingerprint information and determine a second pressing state.

Here, in a process of acquiring a pulse wave, a fingerprint may be regularly acquired and saved as a second fingerprint, and by comparing the second fingerprint with a fingerprint template, a current finger pressing state, i.e., a second pressing state, can be obtained.
S203: Determine whether a finger pressing state change exceeds a preset range;

if not, perform step S204; or if yes, perform step S205.

Specifically, whether a difference between the first pressing state and the second pressing state exceeds a preset range is determined, for example, compared with the first pressing state, whether a pressing position shift, a direction change, or a contact area change of the second pressing state exceeds a preset range is determined; in a case that the difference exceeds the preset range, it indicates that the quality of the acquired pulse wave signal may vary greatly. To ensure the accuracy of health indicator data, it is necessary to stop the process of acquiring a pulse wave that cannot meet a requirement.

S204: Control the pulse wave detection unit to continuously acquire pulse wave data.

Here, specifically, the pulse wave detection unit is controlled to continuously acquire pulse wave data, until the end of acquisition of pulse wave data this time.

S205: Control the pulse wave detection unit to stop acquiring pulse wave data and generate third prompt information.

Here, the third prompt information may be displayed on the display unit of the electronic device to inform the user that the test cannot be completed due to an excessive change in the pressing state (for example: "The finger pressing state has changed and test cannot be completed this time. Please re-test! ")

In response to the first input, the pulse wave detection unit is controlled to acquire the pulse wave data, and in a case that a difference between a finger pressing state of the first user at an end time of acquisition of the pulse wave data and a finger pressing state of the first user at a beginning time of the acquisition of the pulse wave data is less than a preset threshold, the pulse wave data acquired by the pulse wave detection unit is determined as the target pulse wave data.

Here, to ensure the quality of the pulse wave data acquired by the pulse wave detection unit, at the end of the acquisition of the pulse wave data, the fingerprint identification unit acquires fingerprint information of the first user, and obtains the finger pressing state of the first user at this time based on the fingerprint information; in a case that the difference between the finger pressing state and the finger pressing state of the first user at the beginning time of the acquisition of the pulse wave data is less than a preset threshold, it indicates that during the acquisition of the pulse wave data, the finger pressing state of the first user is good, the pulse wave data acquired by the pulse wave detection unit is used as the target pulse wave data, and the quality of the obtained target pulse wave data is high.

Corresponding to the scenario, in an optional implementation, the method of this embodiment of this application may further include:
controlling the pulse wave detection unit to acquire the pulse wave data, and in a case that a difference between a finger pressing state of the first user at an end time of acquisition of the pulse wave data and a finger pressing state of the first user at a beginning time of the acquisition of the pulse wave data is greater than the preset threshold, generating fourth prompt information, where the fourth prompt information is used for prompting the first user to perform biological detection again.

Here, in a case that the difference between the finger pressing state of the first user at the end time of the acquisition of the pulse wave data and the finger pressing state of the first user at the beginning time of the acquisition of the pulse wave data is greater than the preset threshold, it indicates that the finger pressing state of the first user has changed greatly, the fourth prompt information is generated to prompt the first user to perform biological detection again and delete the pulse wave data obtained this time.

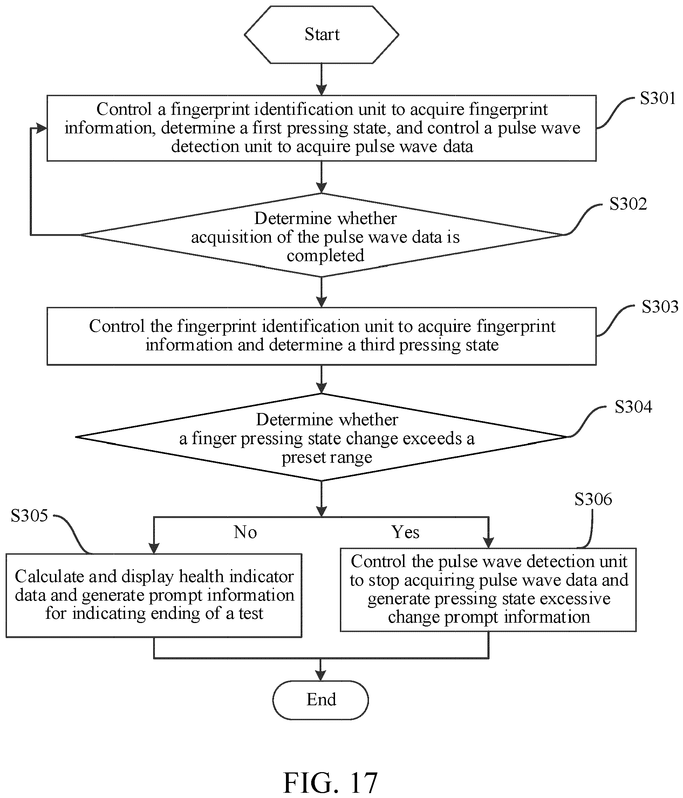

The following is an example corresponding to this scenario, specifically explaining the implementation process of the method in this embodiment of this application, as shown in FIG. 17.

S301: Control a fingerprint identification unit to acquire fingerprint information, determine a first pressing state, and control a pulse wave detection unit to acquire pulse wave data.

S302: Determine whether acquisition of the pulse wave data is completed;
if yes, perform step S303; or if not, return to step S301.

S303: Control the fingerprint identification unit to acquire fingerprint information and determine a third pressing state.

Here, after the acquisition of the pulse wave is completed, a fingerprint may be immediately acquired and saved as a second fingerprint, and by comparing the second fingerprint with a fingerprint template, a pressing state, i.e., the third pressing state, of the finger at the end time of the acquisition of the pulse wave can be obtained.

S304: Determine whether a finger pressing state change exceeds a preset range;
if not, perform step S305; or if yes, perform step S306;

S305: Calculate and display health indicator data and generate prompt information for indicating ending of a test.

S306: Control the pulse wave detection unit to stop acquiring pulse wave data and generate pressing state excessive change prompt information.

Here, compared to the first pressing state, in a case that the pressing position deviation, the direction change, or the contact area change in the third pressing state exceeds the preset range, the terminal may control the pulse wave detection unit to stop acquiring pulse waves and display prompt words, to inform the user of that a test result may be inaccurate due to an excessive change in the pressing state (for example: "The finger pressing state has changed and the test result may be inaccurate. Suggest to re-test!"

By the biological detection control method of this embodiment of this application, by receiving a first input by a first user on a biological detection module, where the biological detection module includes a fingerprint identification unit and a pulse wave detection unit, in response to the first input, obtaining a finger pressing state of the first user obtained by the fingerprint identification unit and pulse wave data acquired by the pulse wave detection unit, determining the pulse wave data as target pulse wave data in a case that the finger pressing state of the first user meets a pulse wave acquisition trigger condition; and obtaining health indicator data of the first user based on the target pulse wave data, a pulse wave detection function can be implemented, thereby improving the pulse wave quality and implementing measurement of health indicators such as a heart rate, blood oxygen, and blood pressure.

It should be noted that the method provided in this embodiment of this application may be executed by a biological detection control apparatus or a control module configured to perform the biological detection control method in the biological detection control apparatus. For example, in this embodiment of this application, the biological detection control apparatus is configured to perform the biological detection control method, to describe the biological detection control apparatus provided in this embodiment of this application.

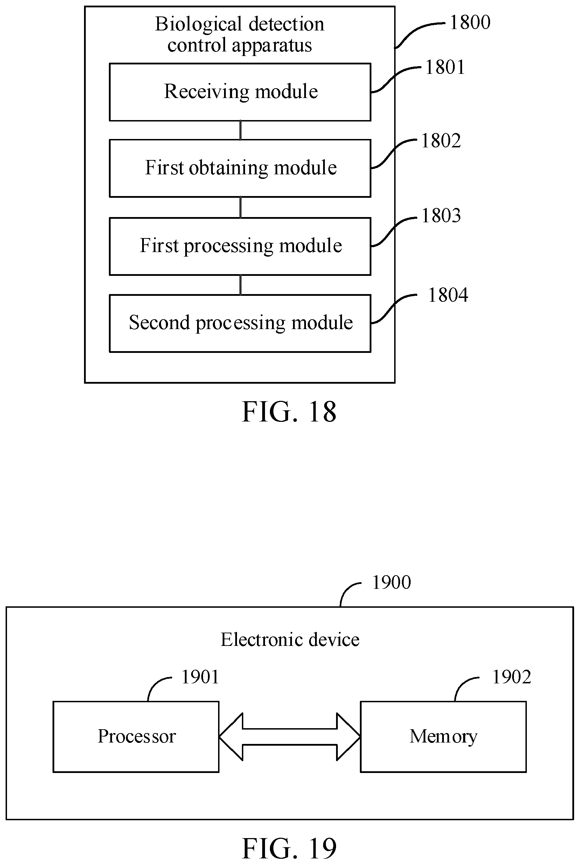

FIG. 18 is a schematic structural diagram of a biological detection control apparatus according to an embodiment of this application. A biological detection control apparatus 1800 may include:
a receiving module 1801, configured to receive a first input by a first user on a biological detection module, where the biological detection module includes a fingerprint identification unit and a pulse wave detection unit;
a first obtaining module 1802, configured to in response to the first input, obtain a finger pressing state of the first user obtained by the fingerprint identification unit and pulse wave data acquired by the pulse wave detection unit;
a first processing module 1803, configured to determine the pulse wave data as target pulse wave data in a case that the finger pressing state of the first user meets a preset condition; and
a second processing module 1804, configured to obtain health indicator data of the first user based on the target pulse wave data.

Optionally, the first processing module 1803 includes one of the following units:
a first processing unit, configured to control the pulse wave detection unit to acquire the pulse wave data and determine the pulse wave data as the target pulse wave data in a case that the finger pressing state of the first user meets a pulse wave acquisition trigger condition;
a second processing unit, configured to control the pulse wave detection unit to acquire the pulse wave data, determine whether the finger pressing state of the first user meets the pulse wave acquisition trigger condition in a process of acquiring the pulse wave data, and in a case that the pulse wave acquisition trigger condition is met, determine the pulse wave data as the target pulse wave data; or
a third processing unit, configured to control the pulse wave detection unit to acquire the pulse wave data, and in a case that a difference between a finger pressing state of the first user at an end time of acquisition of the pulse wave data and a finger pressing state of the first user at a beginning time of the acquisition of the pulse wave data is less than a preset threshold, determine the pulse wave data as the target pulse wave data.

The biological detection control apparatus in this embodiment of this application may be an apparatus, or may be a component, an integrated circuit, or a chip in the terminal. The apparatus may be a mobile electronic device or a non-mobile electronic device. Exemplarily, the mobile electronic device may be a mobile phone, a tablet computer, a notebook computer, a palm computer, an in-vehicle electronic device, a wearable device, an ultra-mobile personal computer (Ultra-Mobile Personal Computer, UMPC), a netbook, or a personal digital assistant (Personal Digital Assistant, PDA); and the non-mobile electronic device may be a network attached storage (Network Attached Storage, NAS), a personal computer (Personal Computer, PC), a television (television, TV), a teller machine, or an automated machine, which are not specifically limited in this embodiment of this application.

The biological detection control apparatus in this embodiment of this application may be an apparatus having an operating system. The operating system may be an Android (Android) operating system, may be an ios operating system, or may be another possible operating system, which is not specifically limited in this embodiment of this application.

The biological detection control apparatus provided in this embodiment of this application can implement the various processes implemented in the method embodiments of FIG. 10 to FIG. 17, details of which are omitted here for brevity.

By the biological detection control apparatus of this embodiment of this application, by receiving a first input by a first user on a biological detection module, where the biological detection module includes a fingerprint identification unit and a pulse wave detection unit, in response to the first input, obtaining a finger pressing state of the first user obtained by the fingerprint identification unit and pulse wave data acquired by the pulse wave detection unit, determining the pulse wave data as target pulse wave data in a case that the finger pressing state of the first user meets a pulse wave acquisition trigger condition, and obtaining health indicator data of the first user based on the target pulse wave data, a pulse wave detection function can be implemented, thereby improving the pulse wave quality and implementing measurement of health indicators such as a heart rate, blood oxygen, and blood pressure.

Optionally, as shown in FIG. 19, an embodiment of this application further provides an electronic device 1900, including a processor 1901, a memory 1902, a program or instructions stored in the memory 1902 and capable of running on the processor 1901. The program or instructions, when executed by the processor 1901, implement the various processes of the biological detection control method embodiments, and in addition, the same technical effect can be achieved, details of which are omitted here for brevity.

It should be noted that the electronic device in this embodiment of this application may be a mobile electronic device or may be a non-mobile electronic device.

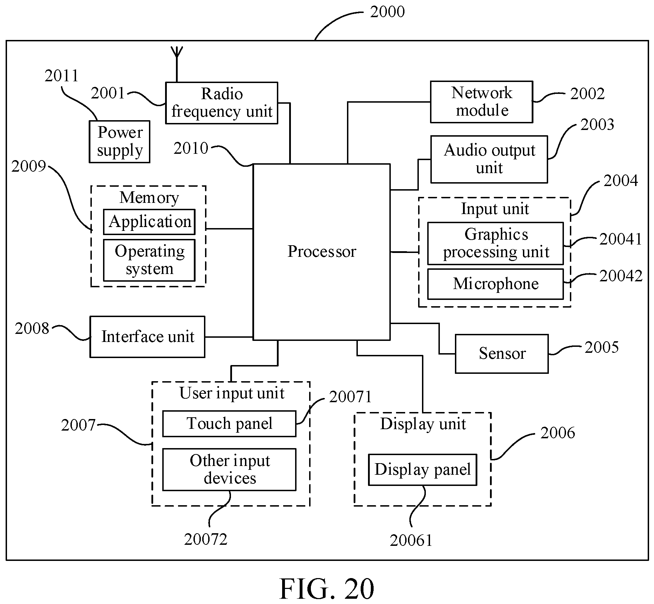

FIG. 20 is a schematic structural diagram of a hardware structure of an electronic device implementing various embodiments of this application.

An electronic device 2000 includes, but is not limited to: components such as a radio frequency unit 2001, a network module 2002, an audio output unit 2003, an input unit 2004, a sensor 2005, a display unit 2006, a user input unit 2007, an interface unit 2008, a memory 2009, a processor 2010, and a power supply 2011.

It may be understood by those skilled in the art that the electronic device 2000 may further include a power supply (for example, a battery) for supplying power to various components, and the power supply may be logically connected to the processor 2010 through a power management system, so as to implement functions such as managing charging or discharging, and power consumption management through the power management system. The structure of the electronic device shown in FIG. 20 constitutes no limitation on the electronic device, and the electronic device may include more or fewer components than those shown in the figure, or some components may be combined, or a different component deployment may be used, which are not described herein again.

The user input unit 2007 is configured to receive a first input by a first user on a biological detection module, where the biological detection module includes a fingerprint identification unit and a pulse wave detection unit. The processor 2010 is configured to: in response to the first input, obtain a finger pressing state of the first user obtained by the fingerprint identification unit and pulse wave data acquired by the pulse wave detection unit; determine the pulse wave data as target pulse wave data in a case that the finger pressing state of the first user meets a preset condition; and obtain health indicator data of the first user based on the target pulse wave data.

In this embodiment of this application, a pulse wave detection function can be implemented, thereby improving the pulse wave quality and implementing measurement of health indicators such as a heart rate, blood oxygen, and blood pressure.

Optionally, the processor 2010 is further configured to perform one of the following steps:
controlling the pulse wave detection unit to acquire the pulse wave data and determining the pulse wave data as the target pulse wave data in a case that the finger pressing state of the first user meets a pulse wave acquisition trigger condition;
controlling the pulse wave detection unit to acquire the pulse wave data, determining whether the finger pressing state of the first user meets the pulse wave acquisition trigger condition in a process of acquiring the pulse wave data, and in a case that the pulse wave acquisition trigger condition is met, determining the pulse wave data as the target pulse wave data; or
controlling the pulse wave detection unit to acquire the pulse wave data, and in a case that a difference between a finger pressing state of the first user at an end time of acquisition of the pulse wave data and a finger pressing state of the first user at a beginning time of the acquisition of the pulse wave data is less than a preset threshold, determining the pulse wave data as the target pulse wave data.

In this embodiment of this application, a pulse wave detection function can be implemented, thereby improving the pulse wave quality and implementing measurement of health indicators such as a heart rate, blood oxygen, and blood pressure.

It should be understood that, in this embodiment of this application, the input unit 2004 may include a graphics processing unit (Graphics Processing Unit, GPU) 20041 and a microphone 20042. The graphics processing unit 20041 performs processing on image data of a static picture or a video that is obtained by an image capturing apparatus (for example, a camera) in a video capture mode or an image capture mode. The display unit 2006 may include a display panel 20061. The display panel 20061 may be configured in a form of a liquid crystal display, an organic light-emitting diode, or the like. The user input unit 2007 includes a touch panel 20071 and other input devices 20072. The touch panel 20071 is also referred to as a touchscreen. The touch panel 20071 may include two parts: a touch detection apparatus and a touch controller. Specifically, the other input devices 20072 may include, but are not limited to, a physical keyboard, a functional key (such as a volume control key or a switch key), a track ball, a mouse, and a joystick, which are not described herein in detail. The memory 2009 may be configured to store software programs and various data, including but not limited to application programs and operating systems. The processor 2010 may integrate an application processor and a modem processor. The application processor mainly processes an operating system, a user interface, an application program, and the like. The modem processor mainly processes wireless communication. It may be understood that the foregoing modem processor may also not be integrated into the processor 2010.

An embodiment of this application further provides a readable storage medium. The readable storage medium may be either non-volatile or volatile. The readable storage medium stores a program or instructions, which, when executed by the processor, implement the various processes of the foregoing biological detection control method embodiments, and in addition, the same technical effect can be achieved, details of which are omitted here for brevity.

The processor is the processor in electronic device described in the foregoing embodiments. The readable storage medium includes a computer-readable storage medium, such as a computer read-only memory (Read-Only Memory, ROM), a random access memory (Random Access Memory, RAM), a disk, an optical disk, or the like.

An embodiment of this application further provides a chip. The chip includes a processor and a communication interface. The communication interface is coupled to the processor. The processor is configured to run a program or instructions to implement the processes of the foregoing biological detection control method embodiments, and in addition, the same technical effect can be achieved, details of which are omitted here for brevity.

It should be understood that the chip mentioned in this embodiment of this application may also be referred to as a system on chip, a system chip, a chip system, or a system-on-a-chip.

It should be noted that the terms "include", "comprise", or any other variation thereof in this specification is intended to cover a non-exclusive inclusion, so that the stated processes, methods, objects, or apparatuses including a series of elements not only include those elements, but also include other elements not explicitly listed, or further include elements inherent to such the processes, methods, objects, or apparatuses. Without more limitations, elements defined by the sentence "including one" does not exclude that there are still other same elements in the processes, methods, objects, or apparatuses. In addition, it should be pointed out that the scope of the method and apparatus in the embodiments of this application is not limited to performing functions in the order shown or discussed, and may also include performing functions in a substantially simultaneous manner or in a reverse order based on the functions involved. For example, the described methods may be performed in an order different from the described order, and various steps may be added, omitted, or combined. In addition, the features described with reference to some examples can be combined in other examples.

According to the descriptions in the foregoing implementations, a person skilled in the art may clearly learn that the method according to the foregoing embodiments may be implemented by relying on software and a commodity hardware platform or by using hardware, and in most cases the former is the preferred. Based on such an understanding, the technical solutions of the present invention essentially, or the part contributing to the prior art, may be presented in the form of a software product. The computer software product is stored in a storage medium (for example, a ROM/RAM, a magnetic disk, or an optical disc) including several instructions to enable a terminal (which may be a mobile phone, a computer, a server, an air conditioner, a network device, or the like) to perform the methods described in the embodiments of this application.

The embodiments of this application have been described above with reference to the accompanying drawings. This application is not limited to the specific embodiments described above, and the specific embodiments described above are merely exemplary and not limitative. Those of ordinary skill in the art may make various variations under the teaching of this application without departing from the spirit of this application and the protection scope of the claims, and such variations shall all fall within the protection scope of this application.

## Claims

1. A biological detection module, comprising:
a packaging layer, on which a light-transmitting part is provided;
a fingerprint identification unit and a pulse wave detection unit disposed within the packaging layer; and
a substrate, the fingerprint identification unit and the pulse wave detection unit being disposed on the substrate and fixedly connected to the substrate, wherein
the pulse wave detection unit directly faces the light-transmitting part.

2. The biological detection module according to claim 1, wherein the pulse wave detection unit comprises a light emitting component and a light receiving component;
the light emitting component and the light receiving component are located on a same side of the fingerprint identification unit; or,
the light emitting component and the light receiving component are respectively located on two opposite sides of the fingerprint identification unit, or,
in a case that the fingerprint identification unit comprises a first fingerprint identification unit and a second fingerprint identification unit, the light emitting component and the light receiving component are located between the first fingerprint identification unit and the second fingerprint identification unit.

3. The biological detection module according to claim 2, wherein in a case that the light emitting component and the light receiving component are located on the same side of the fingerprint identification unit, or in a case that the light emitting component and the light receiving component are located between the first fingerprint identification unit and the second fingerprint identification unit, both transmittance of a first light-transmitting portion and transmittance of a second light-transmitting portion are greater than that of a third light-transmitting portion, wherein
the first light-transmitting portion is a portion where the light-transmitting part corresponds to the light emitting component, the second light-transmitting portion is a portion where the light-transmitting part corresponds to the light receiving component, and the third light-transmitting portion is a portion where the light-transmitting part connects the first light-transmitting portion and the second light-transmitting portion.

4. The biological detection module according to claim 2, wherein in a case that the light emitting component and the light receiving component are respectively located on two opposite sides of the fingerprint identification unit, the light-transmitting part covers the light emitting component, the light receiving component, and the fingerprint identification unit.

5. The biological detection module according to claim 2, wherein in a case that the light emitting component and the light receiving component are respectively located on two opposite sides of the fingerprint identification unit, the light-transmitting part comprises a fourth light-transmitting portion and a fifth light-transmitting portion, the light emitting component directly faces the fourth light-transmitting portion, and the light receiving component directly faces the fifth light-transmitting portion.

6. The biological detection module according to claim 4, wherein both transmittance of a sixth light-transmitting portion and transmittance of a seventh light-transmitting portion are greater than that of an eighth light-transmitting portion, wherein
the sixth light-transmitting portion is a portion where the light-transmitting part corresponds to the light emitting component, the seventh light-transmitting portion is a portion where the light-transmitting part corresponds to the light receiving component and the fingerprint identification unit, and the eighth light-transmitting portion is a portion where the light-transmitting part connects the sixth light-transmitting portion and the seventh light-transmitting portion.

7. The biological detection module according to claim 1, wherein the light-transmitting part is made of a hard optical sheet material; or, the light-transmitting part is provided with an ink coating having light transmittance.

8. The biological detection module according to claim 2, wherein an outer surface, of the light-transmitting part, directly facing the light receiving component is provided with a filter layer, wherein the filter layer is configured to filter out light that enters the light receiving component and exceeds a wavelength band of light to be received by the light receiving component.

9. An electronic device, comprising the biological detection module according to any of claims 1 to 8; and
a housing, the biological detection module being disposed on the housing.

10. A biological detection control method, comprising:
receiving a first input by a first user on a biological detection module, wherein the biological detection module comprises a fingerprint identification unit and a pulse wave detection unit;
in response to the first input, obtaining a finger pressing state of the first user obtained by the fingerprint identification unit and pulse wave data acquired by the pulse wave detection unit;
determining the pulse wave data as target pulse wave data in a case that the finger pressing state of the first user meets a preset condition; and
obtaining health indicator data of the first user based on the target pulse wave data.

11. The method according to claim 10, wherein the determining the pulse wave data as target pulse wave data in a case that the finger pressing state of the first user meets a preset condition comprises one of the following:
controlling the pulse wave detection unit to acquire the pulse wave data and determining the pulse wave data as the target pulse wave data in a case that the finger pressing state of the first user meets a pulse wave acquisition trigger condition;
controlling the pulse wave detection unit to acquire the pulse wave data, determining whether the finger pressing state of the first user meets the pulse wave acquisition trigger condition in a process of acquiring the pulse wave data, and in a case that the pulse wave acquisition trigger condition is met, determining the pulse wave data as the target pulse wave data; or
controlling the pulse wave detection unit to acquire the pulse wave data, and in a case that a difference between a finger pressing state of the first user at an end time of acquisition of the pulse wave data and a finger pressing state of the first user at a beginning time of the acquisition of the pulse wave data is less than a preset threshold, determining the pulse wave data as the target pulse wave data.

12. A biological detection control apparatus, comprising:
a receiving module, configured to receive a first input by a first user on a biological detection module, wherein the biological detection module comprises a fingerprint identification unit and a pulse wave detection unit;
a first obtaining module, configured to in response to the first input, obtain a finger pressing state of the first user obtained by the fingerprint identification unit and pulse wave data acquired by the pulse wave detection unit;
a first processing module, configured to determine the pulse wave data as target pulse wave data in a case that the finger pressing state of the first user meets a preset condition; and
a second processing module, configured to obtain health indicator data of the first user based on the target pulse wave data.

13. The biological detection control apparatus according to claim 12, wherein the first processing module comprises one of the following units:
a first processing unit, configured to control the pulse wave detection unit to acquire the pulse wave data and determine the pulse wave data as the target pulse wave data in a case that the finger pressing state of the first user meets a pulse wave acquisition trigger condition;
a second processing unit, configured to control the pulse wave detection unit to acquire the pulse wave data, determine whether the finger pressing state of the first user meets the pulse wave acquisition trigger condition in a process of acquiring the pulse wave data, and in a case that the pulse wave acquisition trigger condition is met, determine the pulse wave data as the target pulse wave data; or
a third processing unit, configured to control the pulse wave detection unit to acquire the pulse wave data, and in a case that a difference between a finger pressing state of the first user at an end time of acquisition of the pulse wave data and a finger pressing state of the first user at a beginning time of the acquisition of the pulse wave data is less than a preset threshold, determine the pulse wave data as the target pulse wave data.

14. An electronic device, comprising a processor, a memory, and a program or instructions stored on the memory and executable by the processor, wherein the program or instructions, when executed by the processor, implement the steps of the biological detection control method according to any of claims 10 to 11.

15. A readable storage medium, wherein the readable storage medium stores a program or instructions, wherein the program or instructions, when executed by a processor, implement the steps of the biological detection control method according to any of claims 10 to 11.

16. A chip, comprising a processor and a communication interface, wherein the communication interface is coupled to the processor, and the processor is configured to run a program or instructions to implement the steps of the biological detection control method according to any of claims 10 to 11.

17. A computer program product, wherein the computer program product is stored in a non-volatile readable storage medium, and the program product, when executed by at least one processor, implement the steps of the biological detection control method according to any of claims 10 to 11.

18. An electronic device, configured to perform the steps of the biological detection control method according to any of claims 10 to 11.
